(19) Europäisches Patentamt
European Patent Office
Office européen des brevets

(11) Veröffentlichungsnummer: **0 025 864**
**B1**

(12) # EUROPÄISCHE PATENTSCHRIFT

(45) Veröffentlichungstag der Patentschrift: **18.05.83**

(21) Anmeldenummer: **80104863.8**

(22) Anmeldetag: **16.08.80**

(51) Int. Cl.³: **C 07 D 215/26**, C 07 D 401/06, C 07 D 405/06, C 07 D 409/06, A 61 K 31/47

(54) **Tetrahydrochinolinderivate, Verfahren zu ihrer Herstellung, ihre Verwendung und sie enthaltende pharmazeutische Zubereitungen.**

(30) Priorität: **28.08.79 DE 2934609**

(43) Veröffentlichungstag der Anmeldung: **01.04.81 Patentblatt 81/13**

(45) Bekanntmachung des Hinweises auf die Patenterteilung: **18.05.83 Patentblatt 83/20**

(84) Benannte Vertragsstaaten: **AT BE CH DE FR GB IT LI NL SE**

(56) Entgegenhaltungen:

**DE-A-2 362 278**
**FR-A-1 198 123**

**JOURNAL OF MEDICINAL CHEMISTRY, Band 15, Nr. 3, 1972 A.F.CROWTHER et al. «Beta-Adrenergic Blocking Agents 12. Heterocyclic Compounds Related to Propanolol», Seiten 260 bis 266**

**Die Akte enthält technische Angaben, die nach dem Eingang der Anmeldung eingereicht wurden und die nicht in dieser Patentschrift enthalten sind.**

(73) Patentinhaber: **CASSELLA Aktiengesellschaft, Hanauer Landstrasse 526, D-6000 Frankfurt am Main 61 (DE)**

(72) Erfinder: **Gräwinger, Otto, Dr., Hünfelder Strasse 16, D-6000 Frankfurt am Main 61 (DE)**
Erfinder: **Raabe, Thomas, Dr., Schillerstrasse 5, D-6451 Rodenbach (DE)**
Erfinder: **Beyerle, Rudi, Dr., An der Pfaffenmauer 44, D-6000 Frankfurt am Main 60 (DE)**
Erfinder: **Scholtholt, Josef, Prof. Dr., Höhenstrasse 14, D-6450 Mittelbuchen (DE)**
Erfinder: **Nitz, Rolf-Eberhard, Dr., Heinrich-Bingemer-Weg 4, D-6000 Frankfurt am Main 60 (DE)**

(74) Vertreter: **Urbach, Hans-Georg, Dr. et al, Hanauer Landstrasse 526, D-6000 Frankfurt am Main 61 (DE)**

**EP 0 025 864 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Tetrahydrochinolinderivate, Verfahren zu ihrer Herstellung, ihre Verwendung und sie enthaltende pharmazeutische Zubereitungen

Die Erfindung betrifft pharmakologisch wertvolle Derivate des Tetrahydrochinolins der allgemeinen Formel I

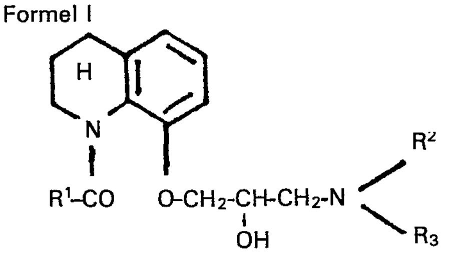

(I)

worin $R^1$ Alkyl mit 1 bis 5 C-Atomen, Thienyl, Furyl, Pyrrolyl, Pyridyl, Phenyl, Phenyl mono-, di- oder trisubstituiert durch Alkyl mit 1 bis 3 C-Atomen, Alkoxy mit 1 bis 3 C-Atomen, Halogen oder Trifluormethyl bedeutet und $R^2$ oder $R^3$ unabhängig voneinander Wasserstoff oder Alkyl mit 1 bis 4 C-Atomen bedeutet oder zusammen mit dem Stickstoffatom, an das sie gebunden sind, 1-Piperidinyl, 1-Piperazinyl, 4-Morpholinyl, 1-Imidazolidinyl oder 1-Pyrrolidinyl bedeuten, deren Säureadditionssalze, ihre Herstellung und Verwendung sowie damit hergestellte pharmazeutische Zubereitungen.

Die Verbindungen der allgemeinen Formel I besitzen in der Alkanolamin-Seitenkette ein asymmetrisches C-Atom und können daher in racemischen und optisch aktiven Formen existieren. Im Rahmen der Erfindung werden unter den Verbindungen der allgemeinen Formel I auch mögliche Stereoisomere und optisch aktive Verbindungen sowie Mischungen davon, insbesondere das Racemat, verstanden.

Die für $R^1$ stehenden Alkylreste besitzen 1 bis 5 C-Atome. Beispiele für $R^1$ stehende Alkylreste sind: Methyl, Äthyl, n-Propyl, iso-Propyl, n-Butyl, iso-Butyl, sec.-Butyl, tert.-Butyl, n-Pentyl, iso-Pentyl. Der für $R^1$ stehende substituierte Phenylrest kann einen, zwei oder drei Substituenten besitzen. Substituenten sind Alkyl- oder Alkoxyreste mit jeweils 1 bis 3 C-Atomen, ferner Halogen, insbesondere Fluor oder Chlor, sowie Tirfluormethyl. Beispiele für geeignete, für $R^1$ stehende substituierte Phenylreste sind 2,3- oder 4-Methyl-, -Äthyl-, -n-Propyl-, oder -iso-Propyl-phenyl, 2,3- oder 4-Methoxy-, -Äthoxy-, -n-Propoxy-phenyl, 3,5-Dimethyl-phenyl, 3,4,5-Trimethyl-phenyl, 3,5-Dimethoxy-phenyl, 3,4,5-Trimethoxy-phenyl, 2,3- oder 4-Chlor- oder -Fluor-phenyl, 2,3- oder 4-Trifluormethyl. Der substituierte Phenylrest besitzt insbesondere einen Alkyl-, Halogen- oder Trifluormethylsubstituenten oder einen, zwei oder drei Alkoxysubstituenten. Für $R^1$ kommen weiters Thienyl, Furyl, Pyrrolyl, vorzugsweise Pyridyl in Betracht.

$R^2$ und $R^3$ bedeuten unabhängig voneinander Wasserstoff oder einen Alkylrest mit 1 bis 4 C-Atomen, der vorzugsweise verzweigt ist. Geeignete Alkylreste sind z.B. Methyl, Äthyl, n-Propyl, n-Butyl, sec.-Butyl, vorzugsweise iso-Propyl, iso-Butyl, tert.-Butyl. $R^2$ und $R^3$ können auch zusammen mit dem Stickstoffatom, an das sie gebunden sind, 1-Piperidinyl, 1-Piperazinyl, 4-Morpholinyl, 1-Imidazolidinyl oder 1-Pyrrolidinyl bedeuten.

Normaleweise werden Verbindungen der allgemeinen Formel I bzw. deren Säureadditionssalze bevorzugt, bei denen $R^2$ für Wasserstoff steht und $R^3$ die Isopropyl- oder tert.-Butylgruppe bedeutet.

Zur Herstellung einer Verbindung der allgemeinen Formel I wird eine Verbindung der allgemeinen Formel II

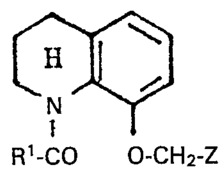

(II)

wobei Z für CH - CH2 oder -CH-CH2-Hal steht und
O OH

Hal ein Halogenatom, insbesondere Chlor oder Brom bedeutet, mit einem Amin der allgemeinen Formel III

$$R^2\text{-NH-}R^3 \qquad (III)$$

umgesetzt und die so erhaltene Verbindung gegebenenfalls mit einer Säure zu einem Säureadditionssalz umgesetzt.

Anstelle einer einheitlichen Verbindung der allgemeinen Formel II kann auch ein Gemisch einer Verbindung der allgemeinen Formel IIa mit einer bezüglich $R^1$ gleich substituierten Verbindung der allgemeinen Formel IIb

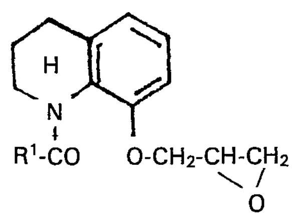

(IIa)

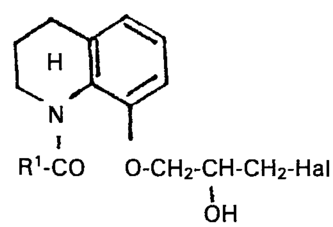

(IIb)

wobei in Formel IIb Hal ein Halogenatom, insbesondere Chlor oder Brom bedeutet, eingesetzt werden.

Die Umsetzung der Verbindungen der allgemeinen Formeln II und III wird normalerweise in einem geeigneten Lösungs- oder Dispersionsmittel durchgeführt, in dem die Reaktionspartner gelöst bzw. suspendiert werden. Solche Lösungs- oder Dispersionsmittel sind z.B. Wasser, aromatische Kohlenwasserstoffe, wie z.B. Benzol, Toluol, Xylol; Ketone wie z.B. Aceton, Methyläthylketon; halogenierte Kohlenwasserstoffe, wie z.B. Chloroform, Tetrachlorkohlenstoff, Chlorbenzol, Methylenchlorid; Äther, wie z.B. Tetrahydrofuran und Dioxan; Sulfoxide, wie z.B. Dimethylsulfoxid; tertiäre Säureamide, wie z.B. Dimethylformamid und N-Methylpyrrolidon. Als Lösungsmittel werden insbesondere polare Lösungsmittel, wie z.B. Alkohole, verwandt. Geeignete Alkohole sind z.B. Methanol, Äthanol, Isopropanol, tert.-Butanol usw. Alkohole mit 1 bis 4 C-Atomen werden bevorzugt. Die Reaktion wird bei Temperaturen von 20°C bis zur Rückflusstemperatur des verwendeten Lösungs- oder Dispergiermittels durchgeführt. Die Reaktion läuft häufig bei Temperaturen von 60 bis 100°C ab. Es kann zweckmässig sein, das Amin der allgemeinen Formel III in einem bis zu 10fach oder gegebenenfalls noch höheren molaren Überschuss einzusetzen und/oder die Reaktionskomponente der allgemeinen Formel II in gelöster bzw. suspendierter Form zu dem gelösten bzw. suspendierten Amin der allgemeinen Formel III zuzugeben. Das Molverhältnis zwischen den Verbindungen der allgemeinen Formeln II und III kann daher 1 : 1 bis 1 : 10 und gegebenenfalls noch mehr betragen. Bei der Anwesenheit einer Verbindung der allgemeinen Formel IIb kann die Umsetzung auch in Gegenwart von säurebindenden Mitteln, wie Pottasche, Soda oder Triäthylamin durchgeführt werden. Ohne säurebindende Mittel erhält man gewöhnlich die Hydrohalogenide der Verbindungen der allgemeinen Formel I.

Zur Bildung von Säureadditionssalzen mit den Verbindungen der allgemeinen Formel I sind anorganische und organische Säuren geeignet. Geeignete Säuren sind beispielsweise Chlorwasserstoff, Bromwasserstoff, Naphthalindisulfonsäure-(1,5), Phosphor-, Salpeter-, Schwefel-, Oxal-, Milch-, Wein-, Essig-, Salicyl-, Benzoe-, Ameisen-, Propion-, Pivalin-, Diäthylessig-, Malon-, Bernstein-, Pimelin-, Fumar-, Malein-, Äpfel-, Sulfamin-, Phenylpropion-, Glucon-, Ascorbin-, Isonicotin-, Nicotin-, Methansulfon-, p-Toluolsulfon-, Citronen- oder Adipinsäure. Pharmazeutisch annehmbare Säureadditionssalze werden bevorzugt. Die Säureadditionssalze können wie üblich durch Vereinigung der Komponenten, zweckmässigerweise in einem geeigneten Verdünnungs- bzw. Dispergiermittel, erhalten werden. Dabei erhält man normalerweise Säureadditionssalze, die pro Mol Base der allgemeinen Formel I 1 Mol Säure als Anion enthalten. Für den Fall, dass $R^1$ einen der genannten Hetarylreste mit einem basischen N-Atom, wie z.B. die Pyridylgruppe, bedeutet und/oder $R^3$ und $R^4$ mit einer weiteren -NH-Gruppe einen der genannten heterocyclischen Ringe bilden, kann die Salzbildung auch zu Säureadditionssalzen führen, die auf 1 Mol Base der allgemeinen Formel I 2 oder 3 Mol Säure als Anion enthalten.

Die Ausgangsverbindungen der allgemeinen Formel II können durch Umsetzung von Verbindungen der allgemeinen Formel IV

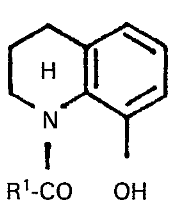

(IV)

mit einem Epihalogenhydrin, zweckmässigerweise mit Epichlorhydrin oder Epibromhydrin, hergestellt werden. Je nach den Reaktionsbedingungen entsteht dabei eine Verbindung der allgemeinen Formel II oder ein Gemisch von Verbindungen der allgemeinen Formeln IIa und IIb. Das entstandene Reaktionsprodukt kann zu seiner weiteren Umsetzung mit der Verbindung III isoliert werden, es kann aber auch ohne Isolierung direkt weiter umgesetzt werden.

Zur Herstellung der Verbindungen der allgemeinen Formel IV wird das 8-Hydroxy-1,2,3,4-tetrahydrochinolin V mit Acylierungsmitteln der allgemeinen Formel VI

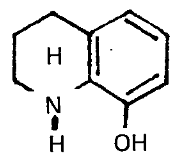

(V) $R^1$-CO-X (VI)

worin X für Halogen, insbesondere Chlor oder Brom, oder den Rest $R^1$-CO-O- steht, umgesetzt.

Optisch aktive Formen der Tetrahydrochinoline der allgemeinen Formel I können durch Trennung der entsprechenden racemischen Tetrahydrochinoline der allgemeinen Formel I mittels üblicher Methoden erhalten werden, z.B. dadurch, dass man das Racemat einer Verbindung der allgemeinen Formel I mit einer optisch aktiven Säure umsetzt, hierauf eine fraktionierte Kristallisation des so erhaltenen diastereomeren Salzgemisches aus einem geeigneten Verdünnungs- oder Lösungsmittel, wie z.B. Äthanol, anschliesst, und schliesslich das optisch aktive Tetrahydrochinolin mit einer Base aus dem Salz in Freiheit setzt. Optisch aktive Verbindungen der allgemeinen Formel I können auch dadurch erhalten werden, dass optisch aktive Ausgangsverbindungen IIb eingesetzt werden. Diese optisch aktiven Ausgangsverbindungen erhält man in bekannter Weise durch Racematspaltung aus den optisch inaktiven Verbindungen IIb.

Die erfindungsgemässen Verbindungen der allgemeinen Formel I und deren pharmazeutisch annehmbaren Säureadditionssalze besitzen wertvolle pharmazeutische Eigenschaften. Insbesondere besitzen sie starke antiarrhythmische blutdrucksenkende Wirkungen. Sie sind daher z.B. für die Behandlung von Herzbeschwerden und Herzkrankheiten, wie Herzarrhythmien, geeignet, ferner zur Behandlung von Hypertension. Die erfindungsgemässen Tetrahydrochinoline können daher am Menschen für sich allein, in Mischung untereinander oder in pharmazeutischen Zubereitungen verabreicht werden, die als aktiven Bestandteil eine wirksame Dosis mindestens eines erfindungsgemässen Tetrahydrochinolins oder eines pharmazeutisch annehmbaren Säureadditionssalzes davon, neben üblichen pharmazeutisch einwandfreien Träger- und Zusatzstoffen enthalten. Geeignete Trägerstoffe sind z.B. Wasser, pflanzliche Öle, Stärke, Gelatine, Milchzucker, Magnesiumstearat, Wachse oder Vaseline. Als Zusatzstoffe können z.B. Netzmittel, Sprengmittel und Konservierungsmittel verwendet werden.

Die pharmazeutischen Präparate können z.B. in

Form von Tabletten, Kapseln, wässrigen oder öligen Lösungen oder Suspensionen, Emulsionen, injizierbaren wässrigen oder öligen Lösungen oder Suspensionen, dispergierbaren Pulvern oder Aerosolmischungen vorliegen. Die pharmazeutischen Präparate können neben den Verbindungen der allgemeinen Formel I auch noch eine oder mehrere andere pharmazeutisch wirksame Substanzen, beispielsweise Beruhigungsmittel, wie z.B. Luminal, Meprobamat, Chlorpromazine und Benzodiazepinsedativa, wie z.B. Diazepam oder Chlordiazepoxid; Vasodilatatoren, wie z.B. Glyzerintrinitrat, Pentaarythrittetranitrat und Carbochromen; Diuretica, wie z.B. Chlorothiazid; das Herz tonisierende Mittel, wie z.B. Digitalis-Präparate; Hypotensionsmittel, wie z.B. Rauwolfia-Alkaloide und Guanethidin; Bronchodilatatoren und sympathomimetische Mittel, wie z.B. Isoprenalin, Ociprenalin, Adrenalin und Ephedrin; α-adrenergische Blockierungsmittel, wie z.B. Phentolamin; β-adrenergische Blockierungsmittel, wie z.B. Propanolol; Kardialmembranstabilisierungsmittel (Antiarrhythmika), wie z.B. Chinidin und Katecholamine, wie Noradrenalin, enthalten.

Die Herstellung der Verbindungen der allgemeinen Formel I wird an folgenden Beispielen näher erläutert:

*Beispiel 1*

1-Nicotinoyl-8-(3-isopropylamino-2-hydroxy-propoxy)-1,2,3,4-tetrahydrochinolin

Zu einer Lösung von 60 ml Isopropylamin in 100 ml Äthanol tropft man unter Rühren bei Raumtemperatur im Verlauf von 30 Minuten eine Lösung von 23 g 1-Nicotinoyl-8-[2,3-epoxy-propoxy(1)]-1,2,3,4-tetrahydrochinolin der Formel

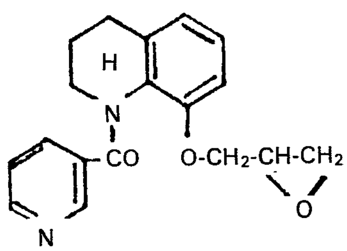

in 100 ml Äthanol unter Rühren zu. Anschliessend wird 8 Stunden unter Rückfluss gekocht, dann die Lösung im Wasserstrahlvakuum eingeengt. Das zurückbleibende Öl wird in 100 ml Essigester gelöst und die Lösung zweimal mit je 30 ml 2n Salzsäure geschüttelt. Die gereinigten salzsauren wässrigen Extrakte werden dann mit 2n Natronlauge auf pH 11 gebracht und zweimal mit je 50 ml Essigester extrahiert. Die gereinigten Essigesterextrakte werden getrocknet und im Wasserstrahlvakuum eingeengt. Es hinterbleibt ein fester Rückstand, der einmal aus Essigester umkristallisiert wird, dann erneut in Essigester gelöst und mit einer Lösung von Naphthalindisulfonsäure-(1,5) im Essigester versetzt wird. Das ausfallende Salz der Naphthalindisulfonsäure-(1,5) wird abgesaugt und zweimal aus Wasser umkristallisiert. Man erhält so das 1-Nicotinoyl-8-[(3-isopropylamino)-2-hydroxy-propoxy]-1,2,3,4-tetrahydrochinolin-naphthalindisulfonat-(1,5) als Monohydrat der Formel

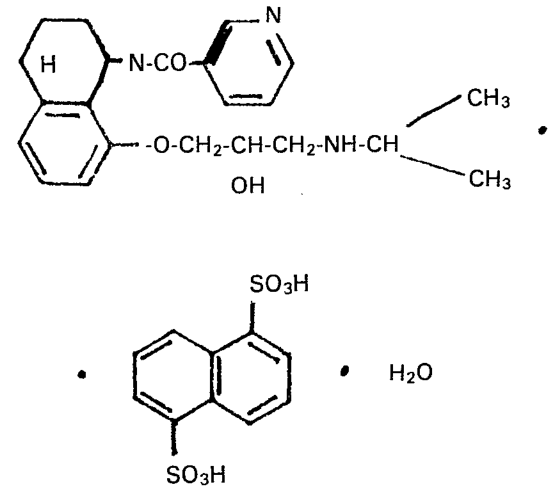

Fp: 292°C (Zers.)
Analyse: ($C_{31}H_{37}N_3O_{10}S_2$)

| | | | | | |
|---|---|---|---|---|---|
| ber.: | C 55,1 | H 5,5 | N 6,2 | O 23,7 | S 9,5 |
| gef.: | C 55,1 | H 5,8 | N 5,9 | O 23,5 | S 9,7 |

Ausbeute: 76% d.Th.

Um die freie Base zu erhalten, wird das Naphthalindisulfonat in Wasser gelöst, die wässrige Lösung mit verdünnter Natronlauge auf pH 10,5 gebracht und die Mischung zweimal mit Essigester extrahiert. Dann werden die Essigesterextrakte im Wasserstrahlvakuum eingeengt und der Rückstand aus Essigester umkristallisiert. Man erhält so das 1-Nicotinoyl-8-[(3-isopropylamino)-2-hydroxy-propoxy]-1,2,3,4-tetrahydrochinolin als freie Base.

Fp: 122°C
Analyse: ($C_{21}H_{27}N_3O_3$)

| | | | | |
|---|---|---|---|---|
| ber.: | C 68,3 | H 7,3 | N 11,4 | O 13,0 |
| gef.: | C 68,2 | H 7,5 | N 11,4 | O 12,9 |

Das als Ausgangsprodukt benutzte 1-Nicotinoyl-8-[(2,3-epoxy-propoxy(1)]-1,2,3,4-tetrahydrochinolin kann wie folgt hergestellt werden:

40 g 1-Nicotinoyl-8-hydroxy-1,2,3,4-tetrahydrochinolin

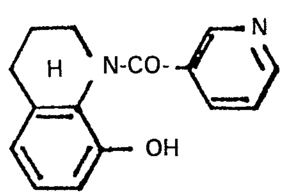

werden in 300 ml Dimethylformamid gelöst und dann bei 5°C unter Rühren 21 g Kalium-tert.-butylat zugesetzt. Man rührt 20 Minuten bei 5°C und tropft dann bei dieser Temperatur unter Rühren 66 g Epichlorhydrin zu, dann lässt man auf Raumtemperatur erwärmen und rührt 20 Stunden nach. Anschliessend wird die Mischung im Wasserstrahlvakuum eingeengt. Das zurückbleibende Öl wird in Wasser/Toluol gelöst, die Toluolphase abgetrennt und zweimal mit verdünnter wässriger Natronlauge und einmal mit Wasser gewaschen, getrocknet und im Wasserstrahlvakuum eingeengt. Es hinterbleibt ein fester

Rückstand, der direkt mit Isopropylamin zum 1-Nicotinoyl-8-[(3-isopropylamino)-2-hydroxy-propoxy]-1,2,3,4-tetrahydrochinolin umgesetzt werden kann. Das als Ausgangsprodukt benutzte 1-Nicotinoyl-8-hydroxy-1,2,3,4-tetrahydrochinolin kann in der üblichen Weise durch Umsetzung des 8-Hydroxy-1,2,3,4-tetrahydrochinolins mit Nicotinsäurechlorid-hydrochlorid in Toluol bei Raumtemperatur in Gegenwart von Triäthylamin erhalten werden. (Fp: Fp: 120°C

*Beispiel 2*

1-Benzoyl-8-(3-morpholin-2-hydroxy-propoxy)-1,2,3,4-tetrahydrochinolin

Zu einer Lösung von 1,7 g Morpholin in 10 ml Äthanol tropft man bei Raumtemperatur eine Lösung von 5 g 1-Benzoyl-8-[2,3-epoxy-propoxy(1)]-1,2,3,4-tetrahydrochinolin in 100 ml Äthanol zu und erhitzt anschliessend 2 Stunden unter Rückfluss. Dann wird im Wasserstrahlvakuum eingeengt. Es hinterbleibt ein Öl, das nach kurzer Zeit fest wird. Nach Umkristallisieren aus Isopropanol erhält man das 1-Benzoyl-8-(3-morpholino-2-hydroxy-propoxy)-1,2,3,4-tetrahydrochinolin der Formel

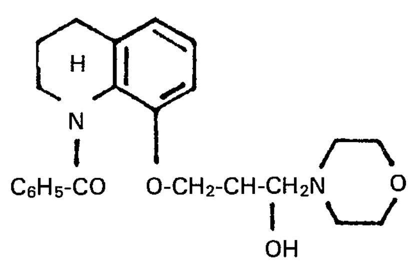

Fp: 120°C

Analyse: ($C_{23}H_{28}N_2O_4$)

| | | | | |
|---|---|---|---|---|
| ber.: | C 69,7 | H 7,1 | N 7,1 | O 16,2 |
| gef.: | C 69,5 | H 7,3 | N 7,2 | O 16,0 |

Ausbeute: 83% d.Th.

Das als Ausgangsprodukt benutzte 1-Benzoyl-8-[2,3-epoxypropoxy(1)]-1,2,3,4-tetrahydrochinolin kann wie folgt hergestellt werden: In einer Lösung von 3 g Natrium-hydroxyd in 350 ml Wasser löst man bei Raumtemperatur 15 g 1-Benzoyl-8-hydroxy-1,2,3,4-tetrahydrochinolin.

Anschliessend werden 6,5 g Epichlorhydrin bei Raumtemperatur zugetropft und die Mischung 24 Stunden bei Raumtemperatur nachgerührt. Dann wird der ausgefallene Niederschlag abgesaugt. Das Produkt kann ohne weitere Reinigung mit Morpholin weiter umgesetzt werden.

Entsprechend den Beispielen 1 bis 2 wurden die in der nachfolgenden Tabelle genannten Verbindungen hergestellt:

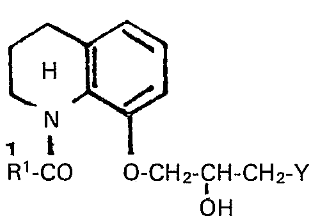

| $R^1$ | Y | Fp. |
|---|---|---|
| 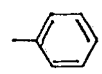 | 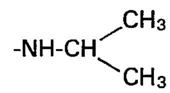 | 155°C (Hydrochlorid) |
| 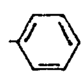 | 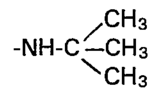 | 163°C |
| 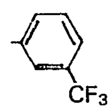 | 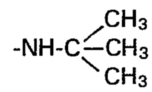 | 116°C |
| 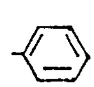 | 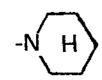 | 110°C |
| $-C_4H_9(n)$ | $-NH_2$ | 95°C |
| 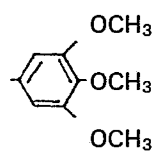 | 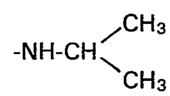 | 215°C (Hydrochlorid) |
| $-CH_3$ | 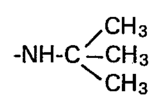 | 198°C (Hydrochlorid) |
| $-C_5H_{11}(n)$ | 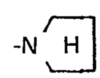 | 121°C |
| 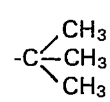 | $-NH-C_4H_9(n)$ | 182°C (Hydrochlorid) |
| 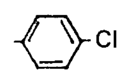 | $-NH-C_2H_5$ | 203°C (Hydrochlorid) |
| 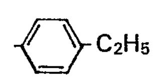 | 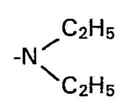 | 186°C (Hydrochlorid) |
| 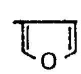 | $-NH-C_3H_7(n)$ | 119°C |
| 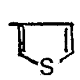 | 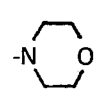 | 194°C (Zers.) (Hydrochlorid) |
| 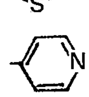 | 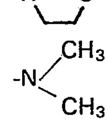 | 128°C |

Die antiarrhythmische Wirkung der erfindungsgemässen Verbindungen wurde an der narkotisierten Ratte und am mit Strophantin vergifteten Hund geprüft.

Methodik für die narkotisierte Ratte:

Männlichen, urethan-narkotisierten Ratten (300 bis 500 g) wurde bei gleichzeitiger Ableitung des EKG Aconitin in einer Dosierung von 5 μ g/kg/min i.v. infundiert. Als Mass für die antiarrhythmische Wirkung wurden die bis zum Auftreten von Extrasystolen, ventrikulären Tachycardien, Kammerflattern und Exitus applizierten Aconitindosen herangezogen. Es wurden die in Tabelle 1 angegebenen Werte erhalten.

Methodik für den mit Strophantin vergifteten Hund:

Nembutal-narkotisierten Hunden wurde bis zum Auftreten stabiler Arrhythmien K-Strophantin (3 μ g/kg/min, 40 min) infundiert. 10 min nach Infusionsende erfolgte die Applikation der Testsubstanzen i.v. bzw. i.d. Normalisierung des EKG für mindestens 10 min wurde als positiver Befund gewertet. Es wurden die in Tabelle 2 angegebenen Werte erhalten.

Tabelle 1

| Substanzen | Aconitinmenge in μg/100 g Ratte bis zum Auftreten von | | | |
|---|---|---|---|---|
| Dosis 10,0 mg/kg i. v. | Extrasystolen | ventrikuläre Tachykardien | ventrikuläres Kammerflattern | Exitus |
| 1-Nicotinoyl-8-(3-isopropyl-amino-2-hydroxy-propoxy)--1,2,3,4-tetrahydrochinolin | 7,0 ± 0,4 | 9,7 ± 0,8 | 12,4 ± 0,8 | 18,0 ± 1,0 |
| Procainamid (Vergleichssubstanz) | 4,0 ± 0,3 | 5,4 ± 0,5 | 7,2 ± 0,3 | 9,0 ± 0,7 |
| Lidocain (Vergleichssubstanz) | 4,3 ± 0,4 | 6,0 ± 0,6 | 8,0 ± 0,9 | 10,4 ± 0,7 |
| Ajmalin (Vergleichssubstanz) | 3,9 ± 0,2 | 5,1 ± 0,4 | 7,0 ± 0,7 | 8,6 ± 0,9 |

Tabelle 2

| Substanzen | ED (mg/kg) i.v. | i.d. |
|---|---|---|
| 1-Nicotinoyl-8-(3-isopropyl-amino-2-hydroxy-propoxy)--1,2,3,4-tetrahydrochinolin | 1,5 | 20 |
| Ajmalin Vergleichssubstanz) | 7,0 | 40 |

Die pharmazeutischen Zubereitungen enthalten beispielsweise 0,1 bis 50 mg, vorzugsweise 0,5 bis 10 mg Wirkstoff/Einheit. Die tägliche Dosierung des Wirkstoffs pro Kilo Körpergewicht beträgt beispielsweise 0,001 bis 1 mg, vorzugsweise 0,005 bis 0,2 mg.

*Beispiel 3*

Zuckerüberzogene Pillen können nach der folgenden Rezeptur hergestellt werden:

| | |
|---|---|
| 1-Nicotinoyl-8-(3-isopropylamino-2-hydroxy-propoxy)-1,2,3,4-tetrahydrochinolin | 1 mg |
| Kornstärke | 100 mg |
| Lactose | 60 mg |
| sec.Calciumphosphat | 30 mg |
| lösliche Stärke | 3 mg |
| Magnesiumstearat | 2 mg |
| kolloidale Kieselsäure | 4 mg |
| | 200 mg |

***Beispiel 4***

Tabletten können nach der folgenden Rezeptur hergestellt werden:

| | |
|---|---|
| 1-Nicotinoyl-8-(3-isopropylamino-2-hydroxy-propoxy)-1,2,3,4-tetrahydrochinolin | 2 mg |
| Lactose | 60 mg |
| Kornstärke | 30 mg |
| lösliche Stärke | 4 mg |
| Magnesiumstearat | 4 mg |
| | 100 mg |

**Patentansprüche für die Vertragsstaaten:**
BE, CH, DE, FR, GB, IT, LI, NL, SE

1. Tetrahydrochinolin der allgemeinen Formel I

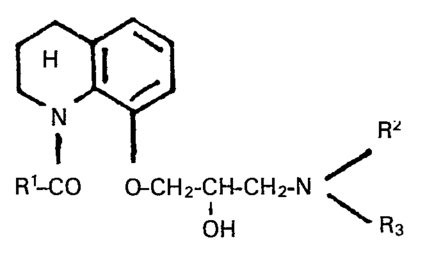

(I)

worin $R^1$ Alkyl mit 1 bis 5 C-Atomen, Thienyl, Furyl, Pyrrolyl, Pyridyl, Phenyl, Phenyl mono-, di- oder trisubstituiert durch Alkyl mit 1 bis 3 C-Atomen, Alkoxy mit 1 bis 3 C-Atomen, Halogen oder Trifluormethyl bedeutet und $R^2$ und $R^3$ unabhängig voneinander Wasserstoff oder Alkyl mit 1 bis 4 C-Atomen bedeuten oder zusammen mit dem Stickstoffatom, an das sie gebunden sind, 1-Piperidinyl, 1-Piperazinyl, 4-Morpholinyl, 1-Imidazolidinyl oder 1-Pyrrolidinyl bedeuten, sowie dessen Säureadditionssalze.

2. Tetrahydrochinolin nach Anspruch 1, dadurch gekennzeichnet, dass ein für $R^1$ stehender Phenylrest durch Chlor oder Fluor substituiert ist.

3. Tetrahydrochinolin nach Anspruch 1 oder 2, dadurch gekennzeichnet, dass $R^2$ Wasserstoff und $R^3$ Isopropyl oder tert.-Butyl bedeuten.

4. 1-Nicotinoyl-8-[(3-isopropylamino)-2-hydroxy-propoxy]-1,2,3,4-tetrahydrochinolin und seine pharmazeutisch annehmbaren Säureadditionssalze.

5. Verfahren zur Herstellung von Verbindungen eines Anspruchs oder mehrerer Ansprüche 1 bis 4, dadurch gekennzeichnet, dass eine Verbindung der allgemeinen Formel II

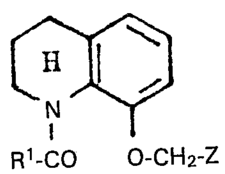

(II)

worin Z für -CH - CH2 oder -CH-CH2-Hal steht und
O OH

Hal Halogen bedeutet, mit einem Amin der allgemeinen Formel III

$R^2$-NH-$R^3$ (III)

wobei $R^1$, $R^2$ und $R^3$ die in den Ansprüchen 1 bis 3 angegebenen Bedeutungen besitzen, umgesetzt und die so erhaltene Verbindung gegebenenfalls mit einer Säure zu einem Säureadditionssalz umgesetzt wird.

6. Verfahren nach Anspruch 5, dadurch gekennzeichnet, dass die Umsetzung der Verbindung der allgemeinen Formel II mit dem Amin der allgemeinen Formel III in einem Lösungs- oder Dispergiermittel bei Temperaturen von 20°C bis zur Rückflusstemperatur des Lösungs- oder Dispergiermittels durchgeführt wird.

7. Pharmazeutische Zubereitung, enthaltend eine wirksame Dosis eines Tetrahydrochinolins der allgemeinen Formel I oder eines pharmazeutisch annehmbaren Säureadditionssalzes davon nach einem oder mehreren der Ansprüche 1 bis 4 neben pharmazeutisch zulässigen Träger- und Zusatzstoffen.

8. Pharmazeutische Zubereitung nach Anspruch 7, enthaltend zusätzlich noch eine oder mehrere pharmazeutisch wirksame Substanzen.

9. Pharmazeutische Zubereitung nach den Ansprüchen 7 oder 8, dadurch gekennzeichnet, dass die weitere pharmazeutisch wirksame Substanz oder Substanzen aus Sedativa, Vasodilatatoren, Diuretica, Hypotensionsmitteln, kardiotonischen Mitteln, α- und/oder β-adrenergischen Blockierungsmitteln, Katecholaminen oder sympathomimetischen Mitteln bestehen.

10. Verwendung eines Tetrahydrochinolins der allgemeinen Formel I oder eines pharmazeutisch annehmbaren Säureadditionssalzes davon nach einem oder mehreren der Ansprüche 1 bis 4 zur Herstellung von Pharmazeutika auf nicht chemischem Wege.

**Patentansprüche für den Vertragsstaat:** AT

1. Verfahren zur Herstellung von Tetrahydrochinolinen der allgemeinen Formel I

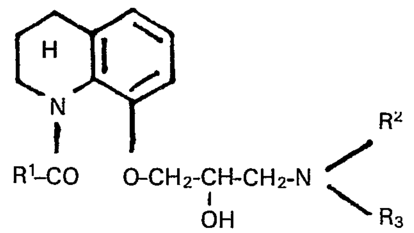

(I)

und ihrer Säureadditionssalze, worin $R^1$ Alkyl mit 1 bis 5 C-Atomen, Thienyl, Furyl, Pyrrolyl, Pyridyl, Phenyl, Phenyl mono-, di- oder trisubstituiert durch Alkyl mit 1 bis 3 C-Atomen, Alkoxy mit 1 bis 3 C-Atomen, Halogen oder Trifluormethyl bedeutet und $R^2$ und $R^3$ unabhängig voneinander Wasserstoff oder Alkyl mit 1 bis 4 C-Atomen bedeuten oder zusammen mit dem Stickstoffatom, an das sie gebunden sind, 1-Piperidinyl, 1-Piperazinyl, 4-Morpholinyl, 1-Imidazolidinyl oder 1-Pyrrolidinyl bedeuten, dadurch gekennzeichnet, dass eine Verbindung der allgemeinen Formel II

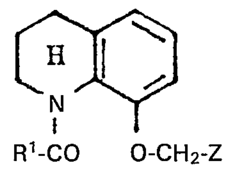

(II)

worin Z für -CH - CH2 oder -CH-CH2-Hal steht und
O OH

Hal Halogen bedeutet, mit einem Amin der allgemeinen Formel III

$R^2$-NH-$R^3$ (III)

umgesetzt und die so erhaltene Verbindung gegebenenfalls mit einer Säure zu einem Säureadditionssalz umgesetzt wird.

2. Verfahren nach Anspruch 1, dadurch gekennzeichnet, dass $R^1$ Chlorphenyl oder Fluorphenyl bedeutet.

3. Verfahren nach Ansprüchen 1 oder 2, dadurch gekennzeichnet, dass $R^2$ Wasserstoff und $R^3$ Isopropyl oder tert.-Butyl bedeuten.

4. Verfahren nach einem oder mehreren der Ansprüche 1 bis 3, dadurch gekennzeichnet, dass $R^1$ 3-Pyridyl, $R^2$ Wasserstoff und $R^3$ Isopropyl bedeuten.

5. Verfahren nach einem oder mehreren der Ansprüche 1 bis 4, dadurch gekennzeichnet, dass die Umsetzung der Verbindung der allgemeinen Formel II mit dem Amin der allgemeinen Formel III in einem Lösungs- oder Dispersionsmittel bei Temperaturen von 20°C bis zur Rückflusstemperatur des Lösungs- oder Dispersionsmittels durchgeführt wird.

6. Verfahren nach einem oder mehreren der Ansprüche 1 bis 5, dadurch gekennzeichnet, dass die Umsetzung der Verbindung der allgemeinen Formel II mit dem Amin der allgemeinen Formel III bei Temperaturen von 60 bis 100°C durchgeführt wird.

7. Verfahren nach einem oder mehreren der Ansprüche 1 bis 6, dadurch gekennzeichnet, dass die Umsetzung der Verbindung der allgemeinen Formel II mit dem Amin der allgemeinen Formel III in Gegenwart eines säurebindenden Mittels durchgeführt wird.

8. Verfahren nach einem oder mehreren der Ansprüche 1 bis 7, dadurch gekennzeichnet, dass anstelle einer einheitlichen Verbindung der allgemeinen Formel II ein Gemisch einer Verbindung der allgemeinen Formel IIa mit einer bezüglich $R^1$ gleich substituierten Verbindung der allgemeinen Formel IIb

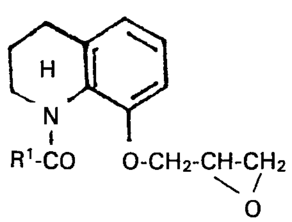

(IIa)

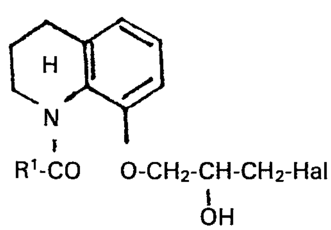

(IIb)

wobei in der allgemeinen Formel IIb Hal ein Halogenatom bedeutet, eingesetzt wird.

9. Verfahren nach einem oder mehreren der Ansprüche 1 bis 8, dadurch gekennzeichnet, dass als Säure für die Überführung der so erhaltenen Verbindung der allgemeinen Formel I in ihr Säureadditionssalz Chlorwasserstoff, Bromwasserstoff, Naphthalinsulfonsäure-(1,5), 'phosphor-, Salpeter-, Schwefel-, Oxal-, Milch-, Wein-, Essig-, Salicyl-, Benzoe-, Ameisen-, Propion-, Pivalin-, Diäthylessig-, Malon-, Bernstein-, Pimelin-, Fumar-, Malein-, Apfel-, Sulfamin-, Phenylpropion-, Glucon-, Ascorbin-, Isonicotin-, Nicotin-, Methansulfon-, p-Toluolsulfon-, Citronen- oder Adipinsäure verwendet wird.

10. Verfahren zur Herstellung von antiarrhythmisch wirksamen Mitteln, dadurch gekennzeichnet, dass eine der nach den Ansprüchen 1 bis 9 erhaltenen Verbindungen durch Mischen mit einem geeigneten pharmazeutisch annehmbaren Trägerstoff in eine zur Verabreichung als antiarrhythmisch wirksames Mittel geeignete Form gebracht wird.

**Claims for the Contracting states:**
BE, CH, DE, FR, GB, IT, LI, NL, SE

1. Tetrahydroquinoline of the general formula I

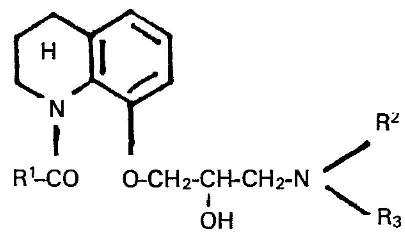

(I)

wherein $R^1$ denotes alkyl with 1 to 5 C atoms, thienyl, furyl, pyrolyl, pyridyl, phenyl or phenyl which is monosubstituted, disubstituted or trisubstituted by alkyl with 1 to 3 C atoms, alkoxy with 1 to 3 C atoms, halogen or trifluormethyl and $R^2$ and $R^3$ independently of one another denote hydrogen or alkyl with 1 to 4 C atoms or, together with the nitrogen atom to which they are bonded, denote 1-piperidinyl, 1-piperazinyl, 4-morpholinyl, 1-imidazolidinyl or 1-pyrrolidinyl, as well as the acid addition salts thereof.

2. Tetrahydroquinoline according to Claim 1, characterised in that $R^1$ is chlorophenyl or fluorophenyl.

3. Tetrahydroquinoline according to Claim 1 or 2, characterised in that $R^2$ denotes hydrogen and $R^3$ denotes isopropyl or tert.-butyl.

4. 1-Nicotinoyl-8-[(3-isopropylamino)-2-hydroxy-propoxy]-1.2.3.4-tetrahydroquinoline and the pharmaceutically acceptable acid addition salts thereof.

5. Process for the preparation of compounds of one of Claims 1 to 4, characterised in that a compound of the general formula II

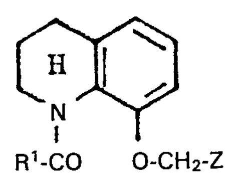

(II)

wherein Z represents 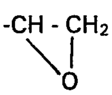 or $-CH-CH_2$-Hal and
OH

Hal denotes halogen, is reacted with an amine of the general formula III

$$R^2\text{-NH-}R^3 \qquad \text{(III)}$$

wherein $R^1$, $R^2$ and $R^3$ have the meanings given in Claims 1 to 3, and, if appropriate, the compound thus obtained is reacted with an acid to give an acid addition salt.

6. The process according to Claim 5, characterised in that the reaction of the compound of the general formula II with the amine of the general formula III is carried out in a solvent or dispersant at temperatures of from 20°C to the reflux temperature of the solvent or dispersant.

7. Pharmaceutical formulation containing an effective dose of a tetrahydroquinoline of the general formula I, or of a pharmaceutically acceptable acid addition salt thereof, according to one or several of Claims 1 to 4, in addition to pharmaceutically permissible excipients and additives.

8. Pharmaceutical formulation according to Claim 7, additionally containing one or more other pharmaceutically active substances.

9. Pharmaceutical formulation according to Claim 7 or 8, characterised in that the other pharmaceutically active substance or substances consist of sedatives, vasodilators, diuretic agents, hypotensive agents, cardiotonic agents, α- and/or β-adrenergic blocking agents, catechol amines or sympathomimetic agents.

10. Use of a tetrahydroquinoline of the general formula I or of a pharmaceutically acceptable salt thereof, according to one or several of Claims 1 to 4, for the preparation of pharmaceuticals by a non-chemical route.

**Revendications pour les Etats contractants:** BE, CH, DE, FR, GB, IT, LI, NL, SE

1. Tétrahydroquinoléine de formule générale I

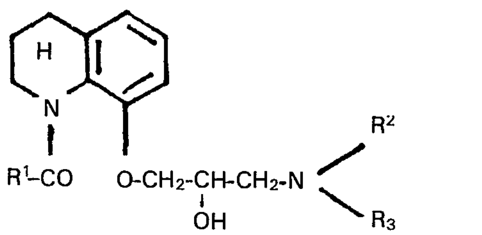

(I)

dans laquelle $R^1$ représente un alkyle en $C_1$-$C_6$, un groupe thiényle, furyle, pyrrolyle ou pyridyle ou encore un phényle portant éventuellement 1, 2 ou 3 substituants choisis parmi des alkyles et des alcoxy en $C_1$ à $C_3$, des halogènes et le groupe trifluorométhyle, et $R^2$ et $R^3$ représentent chacun, indépendamment l'un de l'autre l'hydrogène ou un alkyle en $C_1$ à $C_4$, ou bien forment ensemble et avec l'atome d'azote auquel ils sont liés un radical 1-pipéridinyle, 1-pipérazinyle, 4-morpholinyle, 1-imidazolidinyle ou 1-pyrrolidinyle, ainsi que ses sels d'addition d'acides.

2. Tétrahydroquinoléine selon la revendication 1, caractérisée en ce que $R^1$ est un radical phényle chloré ou fluoré.

3. Tétrahydroquinoléine selon la revendication 1 ou 2, caractérisée en ce que $R^2$ est l'hydrogène et $R^3$ un groupe isopropyle ou tert.-butyle.

4. 1-Nicotinoyl-8-[(3-isopropylamino)-2-hydroxy-propoxy]-1,2,3,4-tétrahydroquinoléine et ses sels d'additions d'acides pharmaceutiquement acceptables.

5. Procédé de préparations des composés selon l'une quelconque des revendications 1 à 4, caractérisé en ce que l'on fait réagir un composé de formule générale II

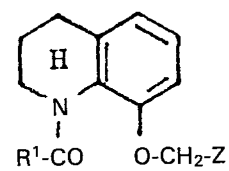

(II)

Z représentant un groupe $-CH-CH_2$ (O) ou $-CH(OH)-CH_2-Hal$,

Hal étant un halogène, avec une amine de formule III

$R^2$-NH-$R^3$ (III)

dans laquelle $R^1$, $R^2$ et $R^3$ ont les significations indiquées dans les revendications 1 à 3, puis on transforme éventuellement le composé ainsi obtenu en un sel d'addition d'acide par réaction avec un acide.

6. Procédé selon la revendication 5, caractérisé en ce que la réaction entre le composé de la formule générale II et l'amine de la formule générale III est effectuée dans un solvant ou un milieu de dispersion à des températures comprises entre 20 °C et la température de reflux du solvant ou du milieu de dispersion.

7. Préparation pharmaceutique contenant une dose efficace d'une tétrahydroquinoléine de la formule générale I ou d'un sel d'addition de celle-ci pharmaceutiquement acceptable selon une ou plusieurs des revendications 1 à 4, avec des véhicules et additifs pharmaceutiquement admissibles.

8. Préparation pharmaceutique selon la revendication 7 contenant en outre une ou plusieus substances douées d'une activité pharmaceutique.

9. Préparation pharmaceutique selon la revendication 7 ou 8 caractérisée en ce que la ou les autres substances douées d'actions pharmaceutiques sont des sédatifs, des vasodilatateurs, des diurétiques, des hypotenseurs, des agents cardiotoniques, des agents de blocage des récepteurs adrénergiques α et/ou β, des catécholamines ou des agents sympathomimétiques.

10. L'utilisation d'une tétrahydroquinoléine de formule générale I ou d'un sel d'addition de celle-ci pharmaceutiquement acceptable selon une ou plusieurs des revendications 1 à 4 pour la préparation de produits pharmaceutiques par voie non chimique.

**Claims for the Contracting state:** AT

1. Process for the preparation of tetrahydroquinolines of the general formula I

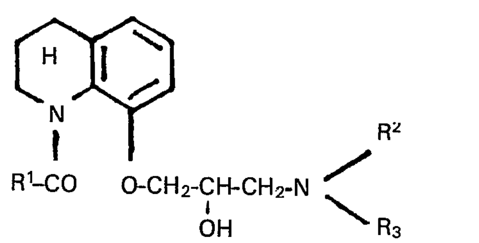

(I)

and the acid addition salts thereof, wherein $R^1$ denotes alkyl with 1 to 5 C atoms, thienyl, furyl, pyrolyl, pyridyl, phenyl or phenyl which is monosubstituted, disubstituted or trisubstituted by alkyl with 1 to 3 C atoms, alkoxy with 1 to 3 C atoms, halogen or trifluoromethyl and $R^2$ and $R^3$ independently of one another denote hydrogen or alkyl with 1 to 4 C atoms or, together with the nitrogen atom to which they are bonded, denote 1-piperidinyl, 1-piperazinyl, 4-morpholinyl, 1-imidazolidinyl or 1-pyrrolidinyl, characterised in that a compound of the general formula II

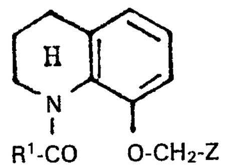

(II)

wherein Z represents 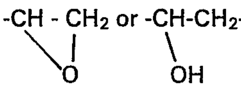 and Hal denotes halogen, is reacted with an amine of the generalformula III

$R^2$-NH-$R^3$ (III)

and if appropriate, the compound thus obtained is reacted with an acid to give an acid addition salt.

2. The process according to Claim 1, characterised in that $R^1$ is chlorophenyl or fluorophenyl.

3. The process according to Claim 1 or 2, characterised in that $R^2$ denotes hydrogen and $R^3$ denotes isopropyl or tert.-buty.

4. The process according to one or several of Claims 1 to 3, characterised in that $R^1$ denotes 3-pyridyl, $R^2$ denotes hydrogen and $R^3$ denotes isopropyl.

5. The process according to one or several of Claims 1 to 4, characterised in that the reaction of the compound of the general formula II with the amine of the general formula III is carried out in a solvent or dispersant at temperatures of from 20°C to the reflux temperature of the solvent or dispersant.

6. The process according to one or several of Claims 1 to 5, characterised in that the reaction of the compound of formula II with the amine of the general formula III is carried out at temperatures of from 60 to 100°C.

7. The process according to one or several of Claims 1 to 6, characterised in that the reaction of the compound of the general formula II with the amine of the general formula III is carried out in the presence of an acid-binding agent.

8. The process according to one or several of Claims 1 to 7, characterised in that, instead of a uniform compound of the general formula II, a mixture of a compound of the general formula IIa with a compound of the general formula IIb

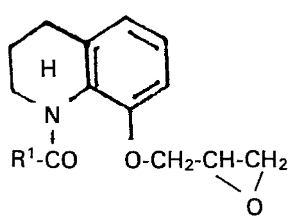

(IIa)

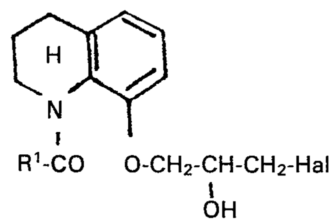

(IIb)

is employed, the latter compound being identically substituted with respect to $R^1$, and Hal in the general formula IIb denoting a halogen atom.

9. The process according to one or several of Claims 1 to 8, characterised in that the acid utilised for converting the compound of the general formula I thus obtained to its addition salt is hydrogen chloride, hydrogen bromide, naphthalene disulphonic acid-(1.5), phosphoric acid, natric acid, sulphuric acid, oxalic acid, lactic acid, tartaric acid, acetic acid, salicylic acid, benzoic acid, formic acid, propionic acid, pivalic acid, diethylacetic acid, malonic acid, succinic acid, pimelic acid, fumaric acid, maleic acid, malic acid, sulphaminic acid, phenylpropionic acid, gluconic acid, ascorbic acid, isonicotinic acid, nicotinic acid, methanesulphonic acid, p-toluenesulphonic acid, citric acid or adipic acid.

10. Process for preparing antiarrhythmically acting agents, characterised in that one of the compounds obtained according to Claims 1 to 9 is converted to a form suitable for administering it as an antiarrhythmically acting agent by mixing with a suitable pharmaceutically acceptable excipient.

**Revendications pour l'etat contractant:** AT

1. Procédé de préparation de tétrahydroquinoléines de formule générale I

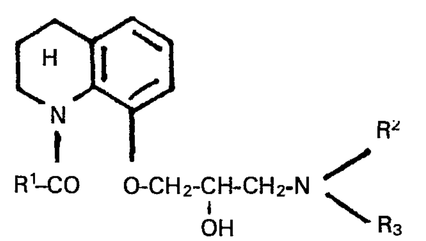

(I)

et de leurs sels d'addition d'acides (formule dans laquelle $R^1$ représente un alkyle en $C_1$ - $C_5$, un groupe thiényle, furyle, pyrrolyle ou pyridyle ou encore un phényle portant éventuellement 1, 2 ou 3 substituants choisis parmi des alkyles et des alcoxy en $C_1$ à $C_3$, des halogènes et le groupe trifluorométhyle, $R^2$ et $R^3$ représentent chacun, indépendamment l'un de l'autre, l'hydrogène ou un alkyle en $C_1$ à $C_4$, ou bien forment ensemble et avec l'atome d'azote auquel ils sont liés un radical 1-imidazolidinyle ou 1-pyrrolidinyle), procédé carac-1-imidazolidinyle ou 1-pyrrolidinyle, procédé caractérisé en ce que l'on fait réagir un composé de formule générale II

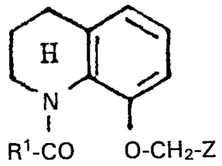

(II)

Z représentant un groupe 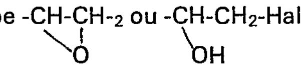

Hal étant un halogène, avec une amine de formule générale III

$R^2-NH-R^3$ (III)

puis on transforme éventuellement le composé ainsi obtenu et un sel d'addition d'acide par réaction avec un acide.

2. Procédé selon la revendication 1, caractérisé en ce que $R^1$ est un radical phényle chloré ou fluoré.

3. Procédé selon la revendication 1 ou 2, caractérisé en ce que $R^2$ est l'hydrogène et $R^3$ un groupe isopropyle ou tert-butyle.

4. Procédé selon une ou plusieurs des revendications 1 à 3, caractérisé en ce que $R^1$ est le radical 3-pyridyle, $R^2$ l'hydrogène et $R^3$ le radical isopropyle.

5. Procédé selon une ou plusieurs des revendications 1 à 4, caractérisé en ce que la réaction entre le composé de formule générale II et l'amine de formule générale III, est effectuée dans un solvant ou un milieu de dispersion à des températures comprises entre 20 °C et la température de reflux du solvant ou du milieu de dispersion.

6. Procédé selon une ou plusieurs des revendications 1 à 5, caractérisé en ce que la réaction entre le composé de formule générale II et l'amine de formule générale III est effectuée entre 60 et 100 °C.

7. Procédé selon une ou plusieurs des revendications 1 à 6, caractérisé en ce que la réaction entre le composé de formule générale II et l'amine de formule générale III est effectuée en présence d'un agent liant les acides.

8. Procédé selon une ou plusieurs des revendications 1 à 7, caractérisé en ce qu'à la place d'un composé défini de formule générale II, on part d'un mélange d'un composé de formule générale IIa et d'un composé de formule générale IIb ayant le même substituant $R^1$

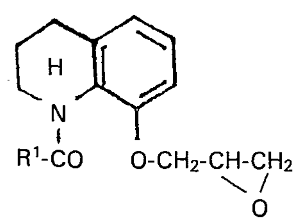

(IIa)

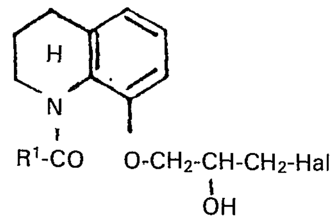

(IIb)

Hal dans la formule générale IIb désignant un atome d'halogène.

9. Procédé selon une ou plusieurs des revendications 1 à 8, caractérisé en ce que pour obtenir le sel d'addition du composé de formule générale I ainsi formé, on utilise comme acide l'acide chlorhydrique, bromhydrique, naphthalène-disulfonique-1,5, phosphorique, nitrique, sulfurique, oxalique, lactique, tartrique, acétique, salicylique, benzoïque, formique, propionique, pivalique, diéthylacétique, malonique, succinique, pimélique, fumarique, maléique, malique, sulfamique, phénylpropionique, gluconique, ascorbique, isonicotinique, nicotinique, méthane-sulfonique, p-toluène-sulfonique, citrique ou adipique.

10. Procédé de préparation d'agents doués d'action antiarythmique, caractérisé en ce que l'on met sous une forme propre à l'administration comme agent doué d'action anti-arythmique un composé obtenu selon l'une quelconque des revendications 1 à 9, par mélange avec un véhicule pharmaceutiquement acceptable.